(19) Europäisches Patentamt
European Patent Office
Office européen des brevets

(11) EP 3 285 730 B1

(12) **EUROPEAN PATENT SPECIFICATION**

(45) Date of publication and mention of the grant of the patent:
**17.06.2020 Bulletin 2020/25**

(21) Application number: **16720282.9**

(22) Date of filing: **22.04.2016**

(51) Int Cl.:
***A61K 8/34*** (2006.01) ***A61Q 5/00*** (2006.01)
***A61Q 5/12*** (2006.01) ***A61K 8/898*** (2006.01)
***A61K 8/04*** (2006.01)

(86) International application number:
**PCT/US2016/028731**

(87) International publication number:
**WO 2016/172403 (27.10.2016 Gazette 2016/43)**

(54) **METHOD OF TREATING HAIR WITH A CONCENTRATED CONDITIONER**

VERFAHREN ZUR HAARBEHANDLUNG MIT EINEM KONZENTRIERTEN HAARSPÜLUNG

MÉTHODE DE TRAITEMENT DES CHEVEUX EN UTILISANT UN APRÈS-SHAMPOING CONCENTRÉ

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**

(30) Priority: **23.04.2015 US 201562151687 P**

(43) Date of publication of application:
**28.02.2018 Bulletin 2018/09**

(73) Proprietor: **The Procter & Gamble Company**
**Cincinnati, OH 45202 (US)**

(72) Inventors:
- **GLENN, Robert, Wayne, Jr.**
**Cincinnati, Ohio 45202 (US)**
- **KAUFMAN, Kathleen, Mary**
**Cincinnati, Ohio 45247 (US)**
- **IWATA, Toshiyuki**
**Singapore, Singapore 138547 (SG)**

(74) Representative: **P&G Patent Germany**
**Procter & Gamble Service GmbH**
**Sulzbacher Straße 40**
**65824 Schwalbach am Taunus (DE)**

(56) References cited:
**EP-A1- 1 329 212**
**WO-A1-2013/176666**
**US-A- 5 077 040**
**US-A1- 2008 292 560**
**US-A1- 2008 292 574**

Note: Within nine months of the publication of the mention of the grant of the European patent in the European Patent Bulletin, any person may give notice to the European Patent Office of opposition to that patent, in accordance with the Implementing Regulations. Notice of opposition shall not be deemed to have been filed until the opposition fee has been paid. (Art. 99(1) European Patent Convention).

EP 3 285 730 B1

## Description

FIELD OF THE INVENTION

Described herein is a method of treating hair with a concentrated hair care composition comprising stearyl alcohol and cetyl alcohol.

BACKGROUND OF THE INVENTION

Today's hair conditioners almost universally comprise high levels of high melting point fatty compounds, the most common of which are C16 to C18 fatty alcohols. These high melting point fatty compounds are employed as structuring agents wherein they are combined with one or more surfactants and an aqueous carrier to form a gel network. The gel network provides a viscous and high yield point rheology which facilitates the dispensing of the conditioner from a bottle or tube and the subsequent distribution and spreading of the product through the hair by the consumer. The gel network structuring also enables incorporation of silicones, perfumes and/or oils in the form of an oil-in-water emulsion that is phase stable. These silicones and/or oils are intended to be deposited onto the hair to provide hair conditioning benefits including wet and dry combing friction reduction and hair manageability.

However, today's gel network hair conditioners can sometimes lead to excessive co-deposits of the high melting point fatty compound on the hair over multiple cycles. Additionally, the high melting point fatty compounds can sometimes build up on hair over multiple cycles and lead to significant waxy build-up on hair and hair weigh down. Indeed, one of the major consumer complaints with many hair conditioners is the creation of a waxy residue which can make the hair look greasy or feel heavy. Many current gel network hair conditioners deposit significantly more high melting point fatty compounds (fatty alcohols) than silicone and/or oil after multiple treatment cycles in technical testing. This is hypothesized to be due to the greater concentration of high melting point fatty compounds in the product relative to the silicone and/or oil. Importantly, such a high level of melting point fatty compounds (fatty alcohols) can be required to produce a shelf stable gel network with sufficient structuring for consumer acceptable viscosity and rheology.

Described herein is a concentrated hair care composition that enables new product opportunities and consumer benefits by addressing the current disadvantages associated with many of today's gel network conditioners. It has been found that concentrated and ultra-low viscosity hair conditioner compositions can be delivered to the hair in foamed form. These new concentrated conditioning compositions enable sufficient dosage from a foam delivery form while also substantially eliminating the need for high melting point fatty compounds or other "insoluble" structurants that can lead to significant co-deposits, build-up, and weigh down of hair. The net result has been an improvement in silicone deposition purity versus many of today's rinse-off products and an improvement in technical performance benefits via the creation of a relatively pure and transparent deposited silicone layer. Additional benefits of the concentrated hair conditioning composition described herein include multicycle hair conditioning without hair weigh down, durable conditioning, reduced hair dye fade, and increased color vibrancy.

Last, complex stability issues can emerge when droplet sizes of an emulsion are driven to the nanoscale. This can be especially problematic in the presence of higher levels of perfume oils required for concentrated products. The concentrated hair care composition described herein can eliminate this issue by providing improved stability.

SUMMARY OF THE INVENTION

Described herein is a method of treating the hair, the method comprising (1) providing a concentrated hair care composition in an aerosol foam dispenser, wherein the concentrated hair care composition comprises (a) from about 4% to about 22% of one or more silicones, by weight of the concentrated hair care composition, wherein the particle size of the one or more silicones is from about 1 nm to about 300 nm; (b) from about 2% to about 6% fatty alcohols, by weight of the concentrated hair care composition, wherein the fatty alcohols are stearyl alcohol and cetyl alcohol, and wherein the ratio of stearyl alcohol to cetyl alcohol is from about 1.5:1 to about 5:1; (c) from about 1% to about 10% propellant, by weight of the concentrated hair care composition; (d) from about 0.5% to about 7% perfume, by weight of the concentrated hair care composition; and (e) from about 75% to about 95% water, by weight of the concentrated hair care composition; wherein the concentrated hair care composition has a liquid phase viscosity of from about 200 centipoise to about 15,000 centipoise; wherein the concentrated hair care composition has silicone to high melting point fatty compound ratio of from about 90:10 to about 30:70; and wherein the concentrated hair care composition has a silicone to perfume ratio of from about 98:2 to about 50:50; (2) dispensing the concentrated hair care composition from the aerosol foam dispenser as a foam; (3) applying the foam to the hair; and (4) rinsing the foam from the hair; wherein the foam has a density of from about 0.025 g/$cm^3$ to about 0.30 g/$cm^3$ when dispensed from the aerosol foam dispenser.

Also described herein is an aerosol foam dispenser comprising a concentrated hair care composition, the concentrated hair care composition comprising (1) from about 4% to about 22% of one or more silicones, by weight of

the concentrated hair care composition, wherein the particle size of the one or more silicones is from about 1 nm to about 300 nm; (2) from about 2% to about 10% fatty alcohols, by weight of the concentrated hair care composition, wherein the fatty alcohols are stearyl alcohol and cetyl alcohol, and wherein the ratio of stearyl alcohol to cetyl alcohol is from about 1.5:1 to about 5:1; (3) from about 1% to about 10% propellant; (4) from about 0.5% to about 7% perfume, by weight of the concentrated hair care composition; and (5) from about 75% to about 95% water, by weight of the concentrated hair care composition; wherein the concentrated hair care composition has a liquid phase viscosity of from about 1 centipoise to about 10,000 centipoise; wherein the concentrated hair care composition has silicone to high melting point fatty compound ratio of from about 100:0 to about 40:60; wherein the concentrated hair care composition has a silicone to perfume ratio of from about 98:2 to about 50:50; wherein the foam has a density of from about 0.025 g/cm$^3$ to about 0.30 g/cm$^3$ when dispensed from the aerosol foam dispenser; and wherein the concentrated hair care composition is rinse-off.

DETAILED DESCRIPTION OF THE INVENTION

While the specification concludes with claims particularly pointing out and distinctly claiming the invention, it is believed that the present invention will be better understood from the following description.

As used herein, the articles including "a" and "an" when used in a claim, are understood to mean one or more of what is claimed or described.

As used herein, "comprising" means that other steps and other ingredients which do not affect the end result can be added. This term encompasses the terms "consisting of" and "consisting essentially of".

As used herein, "mixtures" is meant to include a simple combination of materials and any compounds that may result from their combination.

As used herein, "molecular weight" or "M.Wt." refers to the weight average molecular weight unless otherwise stated.

As used herein, the terms "include," "includes," and "including," are meant to be non-limiting and are understood to mean "comprise," "comprises," and "comprising," respectively.

As used herein, the term "concentrated" means a hair care composition comprising from about 4% to about 20% of one or more silicones, by weight of the hair care composition.

As used herein, the term "nanoemulsion" means an oil-in-water (o/w) emulsion with an average particle size ranging from about 1 nm to about 100 nm. The particle size referred to herein is z-average measured by dynamic light scattering. The nanoemulsion described herein may be prepared by the following methods: (1) mechanically breaking down the emulsion droplet size; (2) spontaneously forming the emulsion (may be referred to as a microemulsion in the literature); and (3) using emulsion polymerization to achieve average particle size in the target range described herein.

All percentages, parts and ratios are based upon the total weight of the compositions of the present invention, unless otherwise specified. All such weights as they pertain to listed ingredients are based on the active level and, therefore, do not include carriers or by-products that may be included in commercially available materials.

Unless otherwise noted, all component or composition levels are in reference to the active portion of that component or composition, and are exclusive of impurities, for example, residual solvents or by-products, which may be present in commercially available sources of such components or compositions.

It should be understood that every maximum numerical limitation given throughout this specification includes every lower numerical limitation, as if such lower numerical limitations were expressly written herein. Every minimum numerical limitation given throughout this specification will include every higher numerical limitation, as if such higher numerical limitations were expressly written herein. Every numerical range given throughout this specification will include every narrower numerical range that falls within such broader numerical range, as if such narrower numerical ranges were all expressly written herein.

Hair Care Composition

The method of treating the hair described herein comprises providing a concentrated hair care composition in an aerosol foam dispenser. The concentrated hair care composition may comprise one or more silicones and perfume.

A. Silicone Deposition Purity

The method of treating hair comprises dispensing the concentrated hair care composition described herein from the aerosol foam dispenser as a dosage of foam. The foam may comprise a silicone deposition purity of from about 40% to about 90%, alternatively from about 42.5% to about 85%, alternatively from about 45% to about 80%, alternatively from about 47.5% to about 75%, and alternatively from about 50% to about 70%, after applying the foam to the hair and rinsing the foam from the hair.

Deposition purity is determined by the ratio of silicone deposited per weight of hair to the total deposition of other ingredients per weight of hair. Silicone is determined by either extraction or digestion of the hair followed by an analysis with a quantitative elemental technique such as ICP for total silicon and converting to silicone based on the % of silicon in the silicone by weight. The total deposition may be determined by the sum of separate deposition measurements or by a Single Inclusive Measurement of total deposition. The separate deposition measurements may include but are not limited to: fatty alcohols, EGDS, quaternized agents, and silicone. Typically these measurements involve extracting the hair then separating the ingredients of interest with chromatography and quantifying with an external calibration based on test solution concentration. The Single Inclusive Measurement of total deposition is gravimetric. The hair is thoroughly extracted and the residue determined by weighing the dissolved residue in the extract after evaporating the solvent. This residue includes both deposited ingredients and naturally occurring extractable compounds from the hair (primarily lipids). The naturally occurring extractable compounds are quantified and subtracted from the total. These include, but are not limited to: fatty acids, squalene, cholesterol, ceramides, wax esters, triglycerides and sterol esters. The method of quantitation is similar to the deposition measurements. Other supporting evidence of Deposition Purity may include spectroscopic or topography mapping of the hair surface.

B. Silicones

The concentrated hair care composition may comprise from about 5% to about 20%, alternatively from about 10% to about 20%, alternatively from about 14% to about 18%, alternatively from about 8% to about 18%, and alternatively from about 10% to about 14% of one or more silicones, by weight of the concentrated hair care composition. In an embodiment, the concentrated hair care composition may comprise from about 4% to about 20%, alternatively from about 4.5% to about 15%, alternatively from about 5% to about 10%, and alternatively from about 5.5% to about 8% of one or more silicones, by weight of the concentrated hair care composition. The particle size of the one or more silicones may be from about 1 nm to about 300 nm, alternatively from about 1 nm to about 100 nm, alternatively from about 5 nm to about 80 nm, alternatively from about 10 nm to about 60 nm, and alternatively from about 12 nm to about 50 nm.

The particle size of the one or more silicones may be measured by dynamic light scattering (DLS). A Malvern Zetasizer Nano ZEN3600 system (*www.malvern.com*) using He-Ne laser 633nm may be used used for the measurement at 25°C.

The autocorrelation function may be analyzed using the Zetasizer Software provided by Malvern Instruments, which determines the effective hydrodynamic radius, using the Stokes-Einstein equation:

$$D = \frac{k_B T}{6\pi\eta R}$$

wherein $k_B$ is the Boltzmann Constant, T is the absolute temperature, $\eta$ is the viscosity of the medium, D is the mean diffusion coefficient of the scattering species, and R is the hydrodynamic radius of particles.

Particle size (i.e. hydrodynamic radius) may be obtained by correlating the observed speckle pattern that arises due to Brownian motion and solving the Stokes-Einstein equation, which relates the particle size to the measured diffusion constant, as is known in the art.

For each sample, three measurements may be made and Z-average values may be reported as the particle size.

In an embodiment, the one or more silicones may be in the form of a nanoemulsion. The nanoemulsion may comprise any silicone suitable for application to the skin and/or hair.

In an embodiment, the one or more silicones may include in their molecular structure polar functional groups such as Si-OH (present in dimethiconols), primary amines, secondary amines, tertiary amines, and quaternary ammonium salts. The one or more silicones may be selected from the group consisting of aminosilicones, pendant quaternary ammonium silicones, terminal quaternary ammonium silicones, amino polyalkylene oxide silicones, quaternary ammonium polyalkylene oxide silicones, and amino morpholino silicones.

The one or more silicones may comprise:

(a) at least one aminosilicone corresponding to formula (V):

$$R'_aG_{3-a}\text{-}Si(OSiG_2)_n\text{-}(OSiG_bR'_{2-b})_m\text{-}O\text{-}SiG_{3-a}\text{-}R'_a \qquad (I)$$

in which:

G is chosen from a hydrogen atom, a phenyl group, OH group, and $C_1$-$C_8$ alkyl groups, for example methyl,
a is an integer ranging from 0 to 3, and in one embodiment a is 0,
b is chosen from 0 and 1, and in one embodiment b is 1,

m and n are numbers such that the sum (n+m) can range for example from 1 to 2 000, such as for example from 50 to 150, wherein n can be for example chosen from numbers ranging from 0 to 1 999, such as for example from 49 to 149, and wherein m can be chosen from numbers ranging for example from 1 to 2 000, such as for example from 1 to 10;

R' is a monovalent group of formula $-C_qH_{2q}L$ in which q is a number from 2 to 8 and L is an optionally quaternized amine group chosen from the groups:

$-NR''-CH_2-CH_2-N'(R^1)_2$,

$-N(R'')_2$,

$-N^+(R'')_3A^-$,

$-N^+H(R'')_2A^-$,

$-N^+H_2(R'')A^-$,

and

$-N(R'')-CH_2-CH_2-N^+R''H_2A^-$,

in which R" can be chosen from a hydrogen atom, phenyl groups, benzyl groups, and saturated monovalent hydrocarbon-based groups, such as for example an alkyl group comprising from 1 to 20 carbon atoms, and $A^-$ is chosen from halide ions such as, for example, fluoride, chloride, bromide and iodide.

In an embodiment, the one or more silicones may include those corresponding to formula (1) wherein a = 0, G=methyl, m and n are numbers such that the sum (n+m) can range for example from 1 to 2 000, such as for example from 50 to 150, wherein n can be for example chosen from numbers ranging from 0 to 1 999, such as for example from 49 to 149, and wherein m can be chosen from numbers ranging for example from 1 to 2 000, such as for example from 1 to 10; and L is $-N(CH_3)_2$ or $-NH_2$, alternatively $-NH_2$.
Additional said at least one aminosilicone of the invention include:
(b) pendant quaternary ammonium silicones of formula (VII):

(VII)

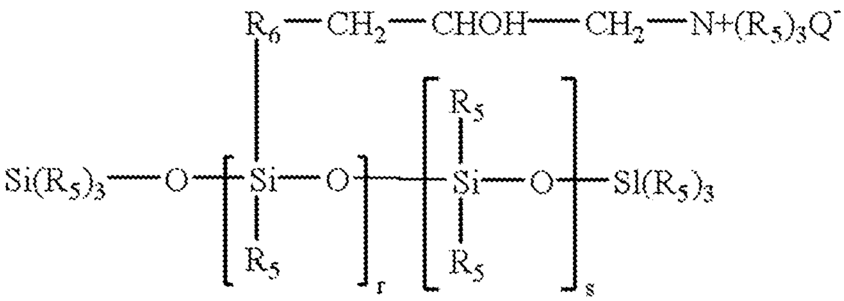

in which:

$R_5$ is chosen from monovalent hydrocarbon-based groups comprising from 1 to 18 carbon atoms, such as $C_1$-$C_{18}$ alkyl groups and $C_2$-$C_{18}$ alkenyl groups, for example methyl;

$R_6$ is chosen from divalent hydrocarbon-based groups, such as divalent $C_1$-$C_{18}$ alkylene groups and divalent $C_1$-$C_{18}$ alkylenoxy groups, for example $C_1$-$C_8$ alkylenoxy groups, wherein said $R_6$ is bonded to the Si by way of an SiC bond;

$Q^-$ is an anion that can be for example chosen from halide ions, such as chloride, and organic acid salts (such as acetate);

r is an average statistical value ranging from 2 to 20, such as from 2 to 8;

s is an average statistical value ranging from 20 to 200, such as from 20 to 50.

Such aminosilicones are described more particularly in U.S. Pat. No. 4,185,087, the disclosure of which is

incorporated by reference herein.

A silicone which falls within this class is the silicone sold by the company Union Carbide under the name "Ucar Silicone ALE 56".

Further examples of said at least one aminosilicone include:

c) quaternary ammonium silicones of formula (VIIb):

(VIIb)

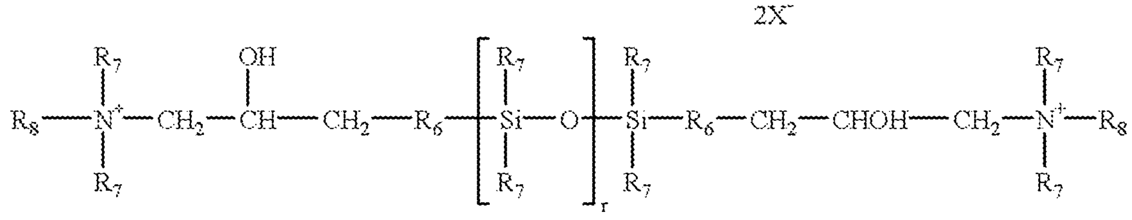

in which:

groups $R_7$, which may be identical or different, are each chosen from monovalent hydrocarbon-based groups comprising from 1 to 18 carbon atoms, such as $C_1$-$C_{18}$ alkyl groups, for example methyl, $C_2$-$C_{18}$ alkenyl groups, and rings comprising 5 or 6 carbon atoms;

$R_6$ is chosen from divalent hydrocarbon-based groups, such as divalent $C_1$-$C_{18}$ alkylene groups and divalent $C_1$-$C_{18}$alkylenoxy, for example $C_1$-$C_8$, group connected to the Si by an SiC bond;

$R_8$, which may be identical or different, represent a hydrogen atom, a monovalent hydrocarbon-based group comprising from 1 to 18 carbon atoms, and in particular a $C_1$-$C_{18}$ alkyl group, a $C_2$-$C_{18}$ alkenyl group or a group $-R_6-NHCOR_7$;

$X^-$ is an anion such as a halide ion, in particular chloride, or an organic acid salt (acetate, etc.);

r represents an average statistical value from 2 to 200 and in particular from 5 to 100.

Such silicones are described, for example, in application EP-A-0 530 974.

Silicones falling within this class are the silicones sold by the company Goldschmidt under the names Abil Quat 3270, Abil Quat 3272 and Abil Quat 3474.

Further examples of said at least one aminosilicone include:

d) quaternary ammonium and polyalkylene oxide silicones wherein the quaternary nitrogen groups are located in the polysiloxane backbone, at the termini, or both.

Such silicones are described in PCT Publication No. WO 2002/010257.

Siliciones falling within this class are the silicones sold by the company Momentive under the names Silsoft Q....

(e) Aminofunctional silicones having morpholino groups of formula (V):

(V)

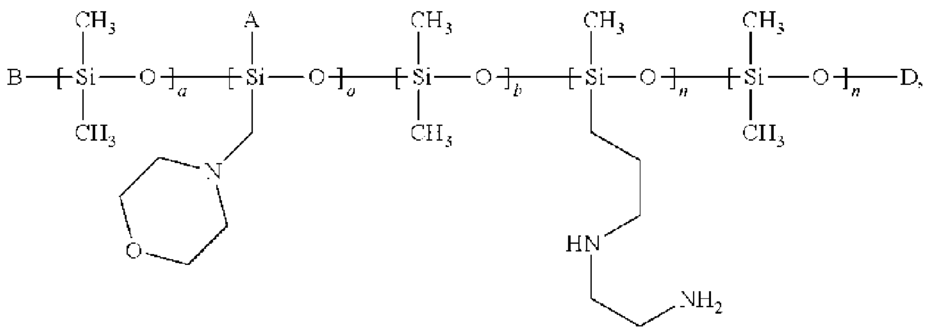

in which

A denotes a structural unit (I), (II), or (III) bound via -O-

(I)

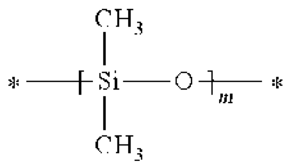

(II)

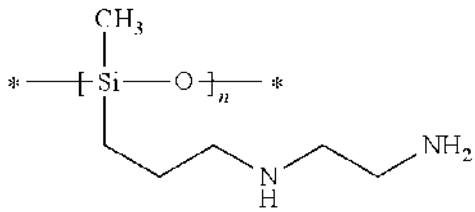

(III)

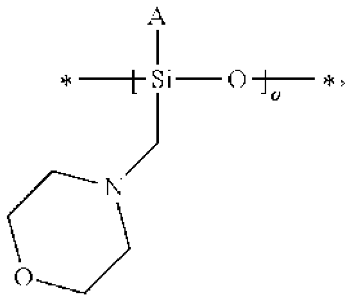

or an oligomeric or polymeric residue, bound via -O-, containing structural units of formulas (I), (II), or (III), or half of a connecting oxygen atom to a structural unit (III), or denotes -OH,

* denotes a bond to one of the structural units (I), (II), or (III), or denotes a terminal group B (Si-bound) or D (O-bound),
B denotes an -OH, $-O-Si(CH_3)_3$, $-O-Si(CH_3)_2OH$, $-O-Si(CH_3)_2OCH_3$ group,
D denotes an -H, $-Si(CH_3)_3$, $-Si(CH_3)_2OH$, $-Si(CH_3)_2OCH_3$ group,
a, b, and c denote integers between 0 and 1000, with the provision that a+b+c>0,
m, n, and o denote integers between 1 and 1000.

Aminofunctional silicones of this kind bear the INCI name: Amodimethicone/Morpholinomethyl Silsesquioxane Copolymer. A particularly suitable amodimethicone is the product having the commercial name Wacker Belsil® ADM 8301E.

Examples of such silicones are available from the following suppliers:

*offered by the company Dow Corning:*

Fluids: 2-8566, AP 6087, AP 6088, DC 8040 Fluid, fluid 8822A DC, DC 8803 & 8813 polymer, 7-6030, AP-8104, AP 8201;
Emulsions: CE-8170 AF Micro Emulsion, 2-8177, 2-8194 Microemulsion, 9224 Emulsion, 939, 949, 959, DC 5-7113 Quat Microemulsion, DC 5-7070 Emulsion, DC CE-8810, CE 8401 Emulsion, CE 1619, Dow Corning Toray SS-3551, Dow Corning Toray SS-3552;

*offered by the company Wacker:*
Wacker Belsil ADM 652, ADM 656, 1100, 1600, 1650 (fluids) ADM 6060 (linear amodimethicone) emulsion; ADM 6057 E (branched amodimethicone) emulsion; ADM 8020 VP (micro emulsion); SLM 28040 (micro emulsion);
*offered by the Company Momentive:*
Silsoft 331, SF1708, SME 253 & 254 (emulsion), SM2125 (emulsion), SM 2658 (emulsion), Silsoft Q (emulsion)
*offered by the company Shin-Etsu:*
KF-889, KF-867S, KF-8004, X-52-2265 (emulsion);

*offered by the Company Siltech Silicones:*
Siltech E-2145, E-Siltech 2145-35;
*offered by the company Evonik Industries:*
Abil T Quat 60th

Some non-limiting examples of aminosilicones include the compounds having the following INCI names: Silicone Quaternium-1, Silicone Quaternium-2, Silicone Quaternium-3, Silicone Quaternium-4, Silicone Quaternium-5, Silicone Quaternium-6, Silicone Quaternium-7, Silicone Quaternium-8, Silicone Quaternium-9, Silicone Quaternium-10, Silicone Quaternium-11, Silicone Quaternium-12, Silicone Quaternium-15, Silicone Quaternium-16, Silicone Quaternium-17, Silicone Quaternium-18, Silicone Quaternium-20, Silicone Quaternium-21, Silicone Quaternium-22, Quaternium-80, as well as Silicone Quaternium-2 Panthenol Succinate and Silicone Quaternium-16/Glycidyl Dimethicone Crosspolymer.

In an embodiment, the aminosilicones can be supplied in the form of a nanoemulsion and include MEM 9049, MEM 8177, MEM 0959, MEM 8194, SME 253, and Silsoft Q.

In an embodiment, the one or more silicones may include dimethicones, and/or dimethiconols. The dimethiconols are hydroxyl terminated dimethylsilicones represented by the general chemical formulas

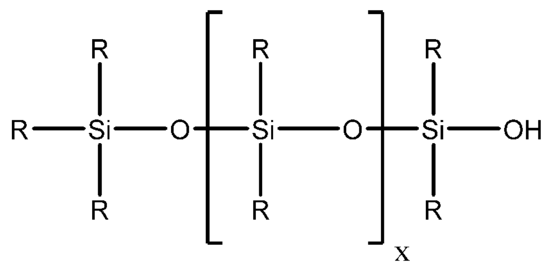

and

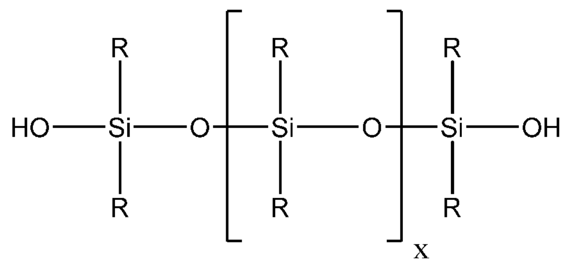

wherein R is an alkyl group (preferably R is methyl or ethyl, more preferably methyl) and x is an integer up to about 500, chosen to achieve the desired molecular weight. Commercial dimethiconols typically are sold as mixtures with dimethicone or cyclomethicone (e.g.,Dow Coming® 1401, 1402, and 1403 fluids).

C. Nonionic Emulsifiers

The concentrated hair care composition may comprise from about 3% to about 20%, alternatively from about 5% to about 15%, and alternatively from about 7.5% to about 12% of a nonionic emulsifier, by weight of the concentrated hair care composition. Nonionic emulsifiers may be broadly defined as including compounds containing an alkylene oxide groups (hydrophilic in nature) with a hydrophobic compound, which may be aliphatic or alkyl aromatic in nature. Examples of nonionic emulsifiers include:

1. Alcohol ethoxylates which are condensation products of aliphatic alcohols having from about 8 to about 18 carbon atoms, in either straight chain or branched chain configuration, with from about 2 to about 35 moles of ethylene oxide, e.g., a coconut alcohol ethylene oxide condensate having from about 2 to about 30 moles of ethylene oxide per mole of coconut alcohol, the coconut alcohol fraction having from about 10 to about 14 carbon atom.
2. The polyethylene oxide condensates of alkyl phenols, e.g., the condensation products of the alkyl phenols having an alkyl group containing from about 6 to about 20 carbon atoms in either a straight chain or branched chain configuration, with ethylene oxide, the said ethylene oxide being present in amounts equal to from about 3 to about 60 moles of ethylene oxide per mole of alkyl phenol.

3. Those derived from the condensation of ethylene oxide with the product resulting from the reaction of propylene oxide and ethylene diamine products.
4. Long chain tertiary amine oxides such as those corresponding to the following general formula: R1 R2 R3 N→O wherein R1 contains an alkyl, alkenyl or monohydroxy alkyl redical of from about 8 to about 18 carbon atoms, from 0 to about 10 ethylene oxide moieties, and from 0 to about 1 glyceryl moiety, and R2 and R3 contain from about 1 to about 3 carbon atoms and from 0 to about 1 hydroxy group, e.g., methyl, ethyl, propyl, hydroxyethyl, or hydroxypropyl radicals (the arrow in the formula represents a semipolar bond).
5. Long chain tertiary phosphine oxides corresponding to the following general formula: RR'R"P→O wherein R contains an alkyl, alkenyl or monohydroxyalkyl radical ranging from about 8 to about 18 carbon atoms in chain length, from 0 to about 10 ethylene oxide moieties and from 0 to about 1 glyceryl moiety and R' and R" are each alkyl or monohydroxyalkyl groups containing from about 1 to about 3 carbon atoms. The arrow in the formula represents a semipolar bond.
6. Long chain dialkyl sulfoxides containing one short chain alkyl or hydroxy alkyl radical of from about 1 to about 3 carbon atoms (usually methyl) and one long hydrophobic chain which include alkyl, alkenyl, hydroxy alkyl, or keto alkyl radicals containing from about 8 to about 20 carbon atoms, from 0 to about 10 ethylene oxide moieties and from 0 to about 1 glyceryl moiety.
7. Polysorbates, e.g., sucrose esters of fatty acids. Such materials are described in U.S. Patent 3,480,616, e.g., sucrose cocoate (a mixture of sucrose esters of a coconut acid, consisting primarily of monoesters, and sold under the tradenames GRILLOTEN LSE 87K from RITA, and CRODESTA SL-40 from Croda).
8. Alkyl polysaccharide nonionic emulsifiers are disclosed in U.S. Patent 4,565,647, Llenado, issued January 21, 1986, having a hydrophobic group containing from about 6 to about 30 carbon atoms, preferably from about 10 to about 16 carbon atoms and a polysaccharide, e.g., a polyglycoside, hydrophilic group. The polysaccharide can contain from about 1.0 to about 10, alternatively from about 1.3 to about 3, and alternatively from about 1.3 to about 2.7 saccharide units. Any reducing saccharide containing 5 or 6 carbon atoms can be used, e.g., glucose, galactose and galactosyl moieties can be substituted for the glucosyl moieties. (Optionally the hydrophobic group is attached at the 2-, 3-, 4-, etc. positions thus giving a glucose or galactose as opposed to a glucoside or galactoside.) The intersaccharide bonds can be, e.g., between the one position of the additional saccharide units and the 2-, 3-, 4-, and/or 6-positions on the preceding saccharide units. Optionally there can be a polyalkyleneoxide chain joining the hydrophobic moiety and the polysaccharide moiety. The alkyl group preferably contains up to about 3 hydroxy groups and/or the polyalkyleneoxide chain can contain up to about 10, preferably less than 5, alkylene moieties. Suitable alkyl polysaccharides are octyl, nonyldecyl, undecyldodecyl, tridecyl, tetradecyl, pentadecyl, hexadecyl, heptadecyl, and octadecyl, di-, tri-, tetra-, penta-, and hexaglucosides, galactosides, lactosides, glucoses, fructosides, fructoses and/or galactoses.
9. Polyethylene glycol (PEG) glyceryl fatty esters, as depicted by the formula RC(O)OCH2 CH(OH)CH2(OCH2CH2)nOH wherein n is from about 5 to about 200, preferably from about 20 to about 100, more preferably from about 30 to about 85, and RC(O)- is an ester wherein R comprises an aliphatic radical having from about 7 to 19 carbon atoms, preferably from about 9 to 17 carbon atoms, more preferably from about 11 to 17 carbon atoms, most preferably from about 11 to 14 carbon atoms. In an embodiment, the combinations of n may be from about 20 to about 100, with C12 -C18, alternatively C12 -C15 fatty esters, for minimized adverse effect on foaming.

In an embodiment, the nonionic emulsifier may be a silicone emulsifier. A wide variety of silicone emulsifiers may be useful herein. These silicone emulsifiers are typically organically modified siloxanes, also known to those skilled in the art as silicone surfactants. Useful silicone emulsifiers include dimethicone copolyols. These materials are polydimethyl siloxanes which have been modified to include polyether side chains such as polyethylene oxide chains, polypropylene oxide chains, mixtures of these chains, and polyether chains containing moieties derived from both ethylene oxide and propylene oxide. Other examples include alkyl-modified dimethicone copolyols, i.e., compounds which contain C2-C30 pendant side chains. Still other useful dimethicone copolyols include materials having various cationic, anionic, amphoteric, and zwitterionic pendant moieties.
In an embodiment, the nonionic emulsifier may have a hydrocarbon chain length of from about 16 to about 20 carbon atoms and from about 20 to about 25 moles of ethoxylate.
In an embodiment, the nonionic emulsifier may have a hydrocarbon chain length of from about 19 to about 11, alternatively from about 9 to about 11 carbon atoms, and from about 2 to about 4 moles of ethoxylate.
In an embodiment, the nonionic emulsifier may comprise a combination of (a) a nonionic emulsifier having a hydrocarbon chain that is branched, has a length of from about 11 to about 15 carbon atoms, and has from about 5 to about 9 moles of ethoxylate; and (b) a nonionic emulsifier having a hydrocarbon chain that has a length of from about 11 to about 13 carbon atoms and has from about 9 to about 12 moles of ethoxylate.
The nanoemulsions used in this invention may be prepared by two different methods: (1) mechanical, and (2) emulsion polymerization.

The first method of preparing the nanoemulsion is the mechanical method in which the nanoemulsion is prepared via the following steps: (1) a primary surfactant is dissolved in water, (2) a silicone is added, and a two-phase mixture is formed, (3) with simple mixing, a co-surfactant is slowly added to the two-phase mixture, until a clear isotropic microemulsion of a siloxane-in-water is formed.

The second method of preparing the nanoemulsion is by emulsion polymerization. Emulsion polymerization methods for making nanoemulsions of polymers involve starting with polymer precursors, i.e., monomers, or reactive oligomers, which are immiscible in water; a surfactant to stabilize polymer precursor droplets in water; and a water soluble polymerization catalyst. Typically, the catalyst is a strong mineral acid such as hydrochloric acid, or a strong alkaline catalyst such as sodium hydroxide. These components are added to water, the mixture is stirred, and polymerization is allowed to advance until the reaction is complete, or the desired degree of polymerization (DP) is reached, and an emulsion of the polymer is formed.

D. Perfume

The concentrated hair care composition may comprise from about 0.5% to about 7%, alternatively from about 1% to about 6%, and alternatively from about 2% to about 5% perfume, by weight of the concentrated hair care composition.

In an embodiment, the concentrated hair care composition may have a silicone to perfume weight ratio of from about 98:2 to about 95:5, alternatively from about 95:5 to about 50:50, alternatively from about 90:10 to 60:40, alternatively from about 85:15 to 70:30.

Examples of suitable perfumes may be provided in the CTFA (Cosmetic, Toiletry and Fragrance Association) 1992 International Buyers Guide, published by CFTA Publications and OPD 1993 Chemicals Buyers Directory 80th Annual Edition, published by Schnell Publishing Co. A plurality of perfume components may be present in the concentrated hair care composition.

E. High Melting Point Fatty Compounds

The concentrated conditioner composition may comprise from about 2% to about 10%, alternatively less than 10%, alternatively less than 8%, alternatively less than 6%, alternatively less than 5%, alternatively less than 4%, alternatively less than 3%, alternatively from about 2% to about 8%, alternatively from about 2% to about 6%, alternatively from about 2% to about 5%, alternatively from about 2% to about 4%, and alternatively from about 2% to about 3% high melting point fatty compounds, by weight of the concentrated hair care composition. In an embodiment, the concentrated hair care composition may have a silicone to high melting point fatty compounds weight ratio of from about 90:10 to about 40:60, alternatively from about 80:20 to about 40:60, alternatively from about 75:25 to about 45:55, and alternatively from about 70:30 to about 50:50.

The high melting point fatty compounds can have a melting point of about 25 °C or higher, and are selected from the group consisting of fatty alcohols, fatty acids, fatty alcohol derivatives, fatty acid derivatives, and mixtures thereof. It is understood by the artisan that high melting point fatty compounds can in some instances fall into more than one classification, e.g., some fatty alcohol derivatives can also be classified as fatty acid derivatives. However, a given classification is not intended to be a limitation on that particular compound, but is done so for convenience of classification and nomenclature. Further, it is understood by the artisan that, depending on the number and position of double bonds, and length and position of the branches, certain compounds having certain required carbon atoms may have a melting point of less than about 25°C. Such compounds of low melting point are not intended to be included in this section. Nonlimiting examples of the high melting point compounds are found in International Cosmetic Ingredient Dictionary, Fifth Edition, 1993, and CTFA Cosmetic Ingredient Handbook, Second Edition, 1992.

The fatty alcohols described herein are those having from about 14 to about 30 carbon atoms, preferably from about 16 to about 22 carbon atoms. These fatty alcohols are saturated and can be straight or branched chain alcohols. Nonlimiting examples of fatty alcohols include cetyl alcohol, stearyl alcohol, behenyl alcohol, and mixtures thereof.

The concentrated hair care composition may comprise from about 2% to about 10%, alternatively from about 3% to about 8%, alternatively from about 4% to about 6% fatty alcohols, by weight of the concentrated hair care composition, wherein the fatty alcohols are stearyl alcohol and cetyl alcohol, and wherein the weight ratio of stearyl alcohol to cetyl alcohol is from about 1.5:1 to about 5:1, alternatively from about 1.75:1 to about 4:1, alternatively from about 2:1 to about 3:1.

The fatty acids useful herein are those having from about 10 to about 30 carbon atoms, preferably from about 12 to about 22 carbon atoms, and more preferably from about 16 to about 22 carbon atoms. These fatty acids are saturated and can be straight or branched chain acids. Also included are diacids, triacids, and other multiple acids which meet the requirements herein. Also included herein are salts of these fatty acids. Nonlimiting examples of fatty acids include lauric acid, palmitic acid, stearic acid, behenic acid, sebacic acid, and mixtures thereof.

The fatty alcohol derivatives and fatty acid derivatives useful herein include alkyl ethers of fatty alcohols, alkoxylated fatty alcohols, alkyl ethers of alkoxylated fatty alcohols, esters of fatty alcohols, fatty acid esters of compounds having esterifiable hydroxy groups, hydroxy- substituted fatty acids, and mixtures thereof. Nonlimiting examples of fatty alcohol derivatives and fatty acid derivatives include materials such as methyl stearyl ether; the ceteth series of compounds such as ceteth-1 through ceteth-45, which are ethylene glycol ethers of cetyl alcohol, wherein the numeric designation indicates the number of ethylene glycol moieties present; the steareth series of compounds such as steareth-1 through steareth- 10, which are ethylene glycol ethers of steareth alcohol, wherein the numeric designation indicates the number of ethylene glycol moieties present; ceteareth 1 through ceteareth-10, which are the ethylene glycol ethers of ceteareth alcohol, i.e., a mixture of fatty alcohols containing predominantly cetyl and stearyl alcohol, wherein the numeric designation indicates the number of ethylene glycol moieties present; C16 -C30 alkyl ethers of the ceteth, steareth, and ceteareth compounds just described; polyoxyethylene ethers of behenyl alcohol; ethyl stearate, cetyl stearate, cetyl palmitate, stearyl stearate, myristyl myristate, polyoxyethylene cetyl ether stearate, polyoxyethylene stearyl ether stearate, polyoxyethylene lauryl ether stearate, ethyleneglycol monostearate, polyoxyethylene monostearate, polyoxyethylene distearate, propyleneglycol monostearate, propyleneglycol distearate, trimethylolpropane distearate, sorbitan stearate, polyglyceryl stearate, glyceryl monostearate, glyceryl distearate, glyceryl tristearate, and mixtures thereof.

In an embodiment, the fatty compound may be a single high melting point compound of high purity. Single compounds of pure fatty alcohols selected may be selected from the group consisting of pure cetyl alcohol, stearyl alcohol, and behenyl alcohol. By "pure" herein, what is meant is that the compound has a purity of at least about 90%, alternatively at least about 95%.

Commercially available high melting point fatty compounds described herein include: cetyl alcohol, stearyl alcohol, and behenyl alcohol having tradenames KONOL series available from Shin Nihon Rika (Osaka, Japan), and NAA series available from NOF (Tokyo, Japan); pure behenyl alcohol having tradename 1-DOCOSANOL available from WAKO (Osaka, Japan), various fatty acids having tradenames NEO-FAT available from Akzo (Chicago, Illinois USA), HYSTRENE available from Witco Corp. (Dublin, Ohio USA), and DERMA available from Vevy (Genova, Italy).

F. Cationic Surfactants

In an embodiment, the concentrated hair care composition may comprise 0%, alternatively from about 0.25% to about 5%, alternatively from about 0.5% to about 4%, and alternatively from about 1% to about 3% cationic surfactants, by weight of the concentrated hair care composition.

The cationic surfactant may be a mono-long alkyl quaternized ammonium salt having the formula (XIII) [from WO2013148778]:

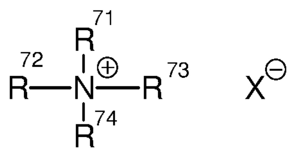

(XIII)

wherein one of $R^{71}$ , $R^{72}$ $R^{73}$ an $R^{74}$ selected from an aliphatic group of from about 14 to about 30 carbon atoms or an aromatic, alkoxy, polyoxyalkylene, alkylamido, hydroxyalkyl, aryl or alkylaryl group having up to about 30 carbon atoms; the remainder of $R^{71}$ , $R^{72}$ $R^{73}$ and $R^{74}$ are independently selected from an aliphatic group of from about 1 to about 8 carbon atoms or an aromatic, alkoxy, polyoxyalkylene, alkylamido, hydroxyalkyl, aryl or alkylaryl group having up to about 8 carbon atoms; and X is a salt-forming anion such as those selected from halogen, (e.g., chloride, bromide), acetate, citrate, lactate, glycolate, phosphate, nitrate, sulfonate, sulfate, alkylsulfate, glutamate, and alkyl sulfonate radicals. The aliphatic groups can contain, in addition to carbon and hydrogen atoms, ether linkages, and other groups such as amino groups. The longer chain aliphatic groups, e.g., those of about 16 carbons, or higher, can be saturated or unsaturated. Preferably, one of $R^{71}$ , $R^{72}$ $R^{73}$ and $R^{74}$ is selected from an alkyl group of from about 14 to about 30 carbon atoms, more preferably from about 16 to about 22 carbon atoms, still more preferably from about 16 to about 18 carbon atoms; the remainder of $R^{71}$, $R^{72}$, $R^{73}$, and $R^{74}$ are independently selected from the group consisting of $CH_3$, $C_2H_5$, $C_2H_4OH$, $CH_2C_5H_5$, and mixtures thereof; and (X) is selected from the group consisting of Cl, Br, $CH_3OSO_3$, and mixtures thereof. It is believed that such mono-long alkyl quatemized ammonium salts can provide improved slippery and slick feel on wet hair.

Nonlimiting examples of such mono-long alkyl quatemized ammonium salt cationic surfactants include: behenyl

trimethyl ammonium chloride available, for example, with tradename Genamine KDMP from Clariant, with tradename INCROQUAT TMC-80 from Croda and ECONOL TM22 from Sanyo Kasei; stearyl trimethyl ammonium chloride available, for example, with tradename CA-2450 from Nikko Chemicals; cetyl trimethyl ammonium chloride available, for example, with tradename CA-2350 from Nikko Chemicals; behenyltrimethylammonium methyl sulfate, available from FeiXiang; hydrogenated tallow alkyl trimethyl ammonium chloride; stearyl dimethyl benzyl ammonium chloride; and stearoyl amidopropyl dimethyl benzyl ammonium chloride.

In an embodiment, the one or more cationic surfactants are those having a shorter alkyl group, i.e., $C_{16}$ alkyl group. Such cationic surfactant includes, for example, cetyl trimethyl ammonim chloride. In an embodiment, cationic surfactants having a shorter alkyl group can be advantageous for improved shelf stability.

In an embodiment, the concentrated hair care composition may comprise an alkyl quat cationic surfactant, wherein the weight ratio of the fatty alcohols to the alkyl quat cationic surfactant is of from about 2.3:1 to about 5:1, alternatively from about 2.5:1 to about 4:1, alternatively from about 2.75:1 to about 3.5:1.

G. Water Miscible Solvents

The concentrated hair care compositions described herein may comprise from about 0.1% to about 25%, alternatively from about 0.1% to about 20%, and alternatively from about 0.1% to about 15% of a water miscible solvent, by weight of the concentrated hair care composition. Non-limiting examples of suitable water miscible solvents include polyols, copolyols, polycarboxylic acids, polyesters and alcohols.

Examples of useful polyols include, but are not limited to, glycerin, diglycerin, propylene glycol, ethylene glycol, butylene glycol, pentylene glycol, 1,3-butylene glycol, cyclohexane dimethanol, hexane diol, polyethylene glycol (200-600), sugar alcohols such as sorbitol, manitol, lactitol and other mono- and polyhydric low molecular weight alcohols (e.g., $C_2$-$C_8$ alcohols); mono di- and oligo-saccharides such as fructose, glucose, sucrose, maltose, lactose, and high fructose corn syrup solids and ascorbic acid.

Examples of polycarboxylic acids include, but are not limited to citric acid, maleic acid, succinic acid, polyacrylic acid, and polymaleic acid.

Examples of suitable polyesters include, but are not limited to, glycerol triacetate, acetylated-monoglyceride, diethyl phthalate, triethyl citrate, tributyl citrate, acetyl triethyl citrate, acetyl tributyl citrate.

Examples of suitable dimethicone copolyols include, but are not limited to, PEG-12 dimethicone, PEG/PPG-18/18 dimethicone, and PPG-12 dimethicone.

Examples of suitable alcohols include, but are not limited to ethanol, n-propanol, isopropanol, n-butanol, sec-butanol, tert-butanol, n-hexanol and cyclohexanol.

Other suitable water miscible solvents include, but are not limited to, alkyl and allyl phthalates; napthalates; lactates (e.g., sodium, ammonium and potassium salts); sorbeth-30; urea; lactic acid; sodium pyrrolidone carboxylic acid (PCA); sodium hyraluronate or hyaluronic acid; soluble collagen; modified protein; monosodium L-glutamate; alpha & beta hydroxyl acids such as glycolic acid, lactic acid, citric acid, maleic acid and salicylic acid; glyceryl polymethacrylate; polymeric plasticizers such as polyquaterniums; proteins and amino acids such as glutamic acid, aspartic acid, and lysine; hydrogen starch hydrolysates; other low molecular weight esters (e.g., esters of $C_2$-$C_{10}$ alcohols and acids); and any other water soluble plasticizer known to one skilled in the art of the foods and plastics industries; and mixtures thereof.

In an embodiment, the water miscible solvents may be selected from the group consisting of glycerin, propylene glycol, dipropylene glycol, and mixtures thereof. EP 0283165 B1 discloses other suitable water miscible solvents, including glycerol derivatives such as propoxylated glycerol.

H. Viscosity Modifiers

The concentrated hair care composition described herein may comprise from about 0.1% to about 2%, alternatively from about 0.1% to about 1%, and alternatively from about 0.1% to about 0.5% of a viscosity modifier, by weight of the concentrated hair care composition. Non-limiting examples of suitable viscosity modifiers include water soluble polymers, cationic water soluble polymers,

Examples of water soluble polymers include, but are not limited to (1) vegetable based polymers such as gum Arabic, tragacanth gum, galactan, guar gum, carob gum, karaya gum, carrageenan, pectin, agar, quince seed, algal colloid, starch (rice, corn, potato, or wheat), and glycyrrhizinic acid; (2) microorganism-based polymers such as xanthan gum, dextran, succinoglucan, and pullulan; and (3) animal-based polymers such as collagen, casein, albumin, and gelatin. Examples of semi-synthetic water-soluble polymers include (1) starch-based polymers such as carboxymethyl starch and methylhydroxypropyl starch; (2) cellulose-based polymers such as methylcellulose, nitrocellulose, ethylcellulose, methylhydroxypropylcellulose, hydroxyethylcellulose, sodium cellulose sulfate, hydroxypropylcellulose, sodium carboxymethylcellulose (CMC), crystalline cellulose, and cellulose powder; and (3) alginate-based polymers such as sodium alginate and propylene glycol alginate. Examples of synthetic water-soluble polymers include (1) vinyl-based

polymers such as polyvinyl alcohol, polyvinyl methyl ether- based polymer, polyvinylpyrrolidone, and carboxyvinyl polymer (CARBOPOL 940, CARBOPOL 941; (2) polyoxyethylene-based polymers such as polyethylene glycol 20,000, polyethylene glycol 6,000, and polyethylene glycol 4,000; (3) copolymer-based polymers such as a copolymer of polyoxyethylene and polyoxypropylene, and PEG/PPG methyl ether; (4) acryl- based polymers such as poly(sodium acrylate), poly(ethyl acrylate), polyacrylamide, polyethylene imines, and cationic polymers. The water-swellable clay minerals are nonionic water-soluble polymers and correspond to one type of colloid-containing aluminum silicate having a triple layer structure. More particular, as examples thereof, mention may be made of bentonite, montmorillonite, beidellite, nontronite, saponite, hectorite, aluminum magnesium silicate, and silicic anhydride.

Examples of cationic water soluble polymers include, but are not limited to (1) quaternary nitrogen-modified polysaccharides such as cation-modified cellulose, cation-modified hydroxyethylcellulose, cation-modified guar gum, cation-modified locust bean gum, and cation-modified starch; (2) dimethyldiallylammonium chloride derivatives such as a copolymer of dimethyldiallylammonium chloride and acrylamide, and poly(dimethylmethylene piperidinium chloride); (3) vinylpyrrolidone derivatives such as a salt of a copolymer of vinylpyrrolidone and dimethylaminoethyl methacrylic acid, a copolymer of vinylpyrrolidone and methacrylamide propyltrimethylammonium chloride, and a copolymer of vinylpyrrolidone and methylvinylimidazolium chloride; and (4) methacrylic acid derivatives such as a copolymer of methacryloylethyldimethylbetaine, methacryloylethyl trimethylammonium chloride and 2-hydroxyethyl methacrylate, a copolymer of methacryloylethyldimethylbetaine, and methacryloylethyl trimethylammonium chloride and methoxy polyethylene glycol methacrylate.

I. Viscosity

The concentrated conditioner composition described herein may have a liquid phase viscosity of from about 200 centipoise to about 15,000 centipoise, alternatively from about 200 centipoise to about 10,000 centipoise, alternatively from about 250 centipoise to about 900 centipoise, and alternatively from about 300 centipoise to about 850 centipoise. In an embodiment, the concentrated care composition described herein may have a liquid phase viscosity of from about 100 centipoise to about 20,000 centipoise, alternatively from about 200 centipoise to about 15,000 centipoise, alternatively from about 300 centipoise to about 10,000 centipoise, alternatively from about 400 centipoise to about 7,500 centipoise, and alternatively from about 600 centipoise to about 5,000 centipoise. The concentrated hair composition viscosity values may be measured using a TA Instruments AR-G2 Rheometer with a concentric cylinder attachment at a shear rate of 100 reciprocal seconds at 25°C.

J. Optional Ingredients

The concentrated hair care composition described herein may optionally comprise one or more additional components known for use in hair care or personal care products, provided that the additional components are physically and chemically compatible with the essential components described herein, or do not otherwise unduly impair product stability, aesthetics or performance. Such optional ingredients are most typically those materials approved for use in cosmetics and that are described in reference books such as the CTFA Cosmetic Ingredient Handbook, Second Edition, The Cosmetic, Toiletries, and Fragrance Association, Inc. 1988, 1992. Individual concentrations of such additional components may range from about 0.001 wt% to about 10 wt% by weight of the conditioning composition.

Emulsifiers suitable as an optional ingredient herein include mono- and di-glycerides, fatty alcohols, polyglycerol esters, propylene glycol esters, sorbitan esters and other emulsifiers known or otherwise commonly used to stabilized air interfaces, as for example those used during preparation of aerated foodstuffs such as cakes and other baked goods and confectionary products, or the stabilization of cosmetics such as hair mousses.

Further non-limiting examples of such optional ingredients include preservatives, perfumes or fragrances, cationic polymers, viscosity modifiers, coloring agents or dyes, conditioning agents, hair bleaching agents, thickeners, moisturizers, foam boosters, additional surfactants or nonionic cosurfactants, emollients, pharmaceutical actives, vitamins or nutrients, sunscreens, deodorants, sensates, plant extracts, nutrients, astringents, cosmetic particles, absorbent particles, adhesive particles, hair fixatives, fibers, reactive agents, skin lightening agents, skin tanning agents, antidandruff agents, perfumes, exfoliating agents, acids, bases, humectants, enzymes, suspending agents, pH modifiers, hair colorants, hair perming agents, pigment particles, anti-acne agents, antimicrobial agents, sunscreens, tanning agents, exfoliation particles, hair growth or restorer agents, insect repellents, shaving lotion agents, non-volatile solvents or diluents (water-soluble and water-insoluble), co-solvents or other additional solvents, and similar other materials.

In an embodiment, the optional ingredients include anti-dandruff agents which may be selected from: pyridinethione salts, azoles (e.g.,ketoconazole, econazole, and elubiol), selenium sulfide, particulate sulfur, salicylic acid, and mixtures thereof. A typical anti-dandruff agent is pyridinethione salt. Hair care compositions can also include a zinc-containing layered material. An example of a zinc-containing layered material can include zinc carbonate materials. Of these, zinc carbonate and pyridinethione salts (particularly zinc pyridinethione or "ZPT) are common in the composition,

and often present together.

K. Aerosol Foam Dispenser

The aerosol foam dispenser may comprise a reservoir for holding the concentrated hair treatment composition. The reservoir may be made out of any suitable material selected from the group consisting of plastic, metal, alloy, laminate, and combinations thereof. In an embodiment, the reservoir may be for one-time use. In an embodiment, the reservoir may be removable from the aerosol foam dispenser. Alternatively, the reservoir may be integrated with the aerosol foam dispenser. In an embodiment, there may be two or more reservoirs.

In an embodiment, the reservoir may be comprised of a material selected from the group consisting of rigid materials, flexible materials, and combinations thereof. The reservoir may be comprised of a rigid material if it does not collapse under external atmospheric pressure when it is subject to an interior partial vacuum.

The foam may have a density of from about 0.025 $g/cm^3$ to about 0.25 g/cm, alternatively from about 0.05 $g/cm^3$ to about 0.20 g/cm, and alternatively from about 0.075 $g/cm^3$ to about 0.15 g/cm when dispensed from the aerosol foam dispenser. In an embodiment, the foam may have a density of from about 0.025 $g/cm^3$ to about 0.30 $g/cm^3$, alternatively from about 0.035 $g/cm^3$ to about 0.20 $g/cm^3$, alternatively from about 0.05 $g/cm^3$ to about 0.15 $g/cm^3$, and alternatively from about 0.075 $g/cm^3$ to about 0.12 $g/cm^3$.

L. Propellant

The concentrated hair care composition described herein may comprise from about from about 1% to about 10% propellant, alternatively from about 1% to about 6% propellant, alternatively from about 2% to about 5% propellant, and alternatively from about 3% to about 4% propellant, by weight of the concentrated hair care composition.

The propellant may comprise one or more volatile materials, which in a gaseous state, may carry the other components of the concentrated hair care composition in particulate or droplet form. The propellant may have a boiling point within the range of from about -45° C. to about 5° C. The propellant may be liquefied when packaged in convention aerosol containers under pressure. The rapid boiling of the propellant upon leaving the aerosol foam dispenser may aid in the atomization of the other components of the concentrated hair care composition.

Aerosol propellants which may be employed in the aerosol composition may include the chemically-inert hydrocarbons such as propane, n-butane, isobutane, cyclopropane, and mixtures thereof, as well as halogenated hydrocarbons such as dichlorodifluoromethane, 1,1-dichloro-1,1,2,2-tetratluoroethane, 1-chloro-1,1 -difluoro-2,2-trifluoroethane, 1-chloro-1,1-difluoroethylene, 1,1-difluoroethane, dimethyl ether, monochlorodifluoromethane, trans-1,3,3,3-tetrafluoropropene, and mixtures thereof. The propellant may comprise hydrocarbons such as isobutane, propane, and butane-these materials may be used for their low ozone reactivity and may be used as individual components where their vapor pressures at 21.1° C. range from about 1.17 Bar to about 7.45 Bar, alternatively from about 1.17 Bar to about 4.83 Bar, and alternatively from about 2.14 Bar to about 3.79 Bar.

Method of Treating Hair

The method of treating the hair described herein comprises (1) providing a concentrated hair care composition, as described herein, in an aerosol foam dispenser, (2) dispensing the concentrated hair care composition from the aerosol foam dispenser as a dosage of foam; (3) applying the foam to the hair; and (4) rinsing the foam from the hair.

EXAMPLES

The following examples illustrate embodiments of the concentrated hair care composition described herein. The exemplified compositions can be prepared by conventional formulation and mixing techniques. It will be appreciated that other modifications of the concentrated hair care composition within the skill of those in the shampoo formulation art can be undertaken without departing from the spirit and scope of this invention. All parts, percentages, and ratios herein are by weight unless otherwise specified. Some components may come from suppliers as dilute solutions. The amount stated reflects the weight percent of the active material, unless otherwise specified.

The following are non-limiting examples of the concentrated hair care composition described herein.

Formulations:

Distilled water and the aminosilicone are mixed using an overhead mixer at 100-150 rpm into a stainless steel beaker. The cetyl alcohol and stearyl alcohol are added into the beaker and the mixture is heated to 70-75°C. Cetyltrimethylammonium choloride is then added and mixing speed is increased to 250-350 rpm. When the materials are melted,

the mixture is cooled to 35°C under stirring. The perfume and Kathon are added into the mixture and stirred for approximately 10 minutes. In the case of aerosol compositions, the mixture is transferred to appropriate container and propellant Aeron-46 is added.

Conditioner Compositions

| Raw Material | Ex. 1 | Ex. 2 | Ex. 3 | Ex. 4 | Ex. 5 |
|---|---|---|---|---|---|
| | Wt (%) | Wt (%) | Wt (%) | Wt (%) | Wt (%) |
| Aminopropylaminoethylpolysiloxane[1] | 12.00% | 12.00% | 12.00% | 16.0% | 15.04% |
| Cetyl Alcohol | 3.00% | 1.80% | 1.80% | 1.35% | 1.27% |
| Stearyl Alcohol | 3.00% | 4.20% | 4.20% | 3.15% | 2.96% |
| Perfume | 3.00% | 3.00% | 3.00% | 3.00% | 3.00% |
| Cetyltrimethylammonium chloride[2] | 2.50% | | 1.60% | 2.50% | 2.40% |
| Steartrimonium chloride[3] | | 2.5% | | | |
| Methylchloroisothiazolinone / Methylisothiazolinone[4] | 0.0005% | 0.0005% | 0.0005% | 0.0005% | 0.0005% |
| Propellant A46[5] | | | | | 6.00% |
| Destilled water | Q.S. | Q.S. | Q.S. | Q.S. | Q.S. |
| Ratio of Stearyl Alcohol: Cetyl Alcohol | 1 | 2.33 | 2.33 | 2.33 | 2.33 |
| Ratio of Fatty Alcohol: Quat | 2.4 | 2.40 | 3.75 | 1.80 | 1.80 |

1. Silsoft 253 Microemulsion supplied by Momentive
2. CTAC (at active level of 25%) supplied by Aldrich
3. Genamin STAC supplied by Clariant
4. Kathon CG available from Dow (1.5 wt% active)
5. Isobutane/Propane 84.85/15.15; sipplied by Diversified CPC

TEST METHODS

Wet & Dry Conditioning Performance Measurements

1. Virgin brown hair switches (20 gram in weight and 10 inches in length) are wet with water of 100°F at a sink with a shower head fixture (flow rate is 1.5 gallons per minute) for 15 to 20 seconds.
2. An amount of 2mL of a clarifying shampoo (Pantene Pro-V Purifying Shampoo) is added via a syringe and milked/scrubbed for 30 seconds followed by a 30 seconds rinse using the shower head (with gentle manipulation at top of switch to ensure uniform rinsing).
3. An amount of 0.67 grams of the concentrated conditioner product is applied evenly over the hair switch via a syringe and milked/scrubbed for 30 seconds followed by a 30 seconds rinse using the shower head (with gentle manipulation at top of switch to ensure uniform rinsing).

During step 3, the treatment operator applies the concentrated conditioner (blinded to the treatments) wearing gloves. The operator evaluates the in-use and wet conditioning properties (smooth feel while applying, smooth feel while rinsing, wet detangling/combing, and smooth feel after rinsing). Each attribute is rated on a 1 to 10 scale with 10 being the highest conditioning performance and 1 being the least. Afterwards, the hair is then air dried overnight while hanging and the treatment operator evaluates the dry conditioning properties without gloves (moisturized feel, soft feel, dry combing and hair volume).

Viscosity Measurements

Viscosity is measured using a Brookfield R/S Plus Viscometer with a C75 spindle at 25°C and at a shear rate of 2 1/s. An amount of 2.5 ml of the product was dispensed into viscometer via a syringe. The steady state viscosity is recorded after a period of 180 seconds.

DATA

The following data demonstrates improved wet conditioning (wet detangling/combing and smooth after rinsing) and improved dry conditioning (moisturized feel, soft feel and dry combing) with increasing the ratio of stearyl alcohol to cetyl alcohol from 1:1 to 2.33:1. Such strong wet and dry conditioning performance was demonstrated with two differing alkyl quat surfactants (CTAC and STAC) and at three differing fatty alcohol:quat ratios (1.8:1, 2.4:1 and 3.75:1). Increasing the aminosilicone level from 12% to 16% further improves the dry conditioning performance (moisturized feel and dry combing).

Table 1

| | Conditioner - Wet Properties | |
|---|---|---|
| | Wet Detangling/ Combing | Smooth Feel AFTER rinsing |
| Example 1 | 8.5 | 8 |
| Example 2 | 9.5 | 9.3 |
| Example 3 | 9.3 | 9.3 |
| Example 4 | 9.5 | 9.5 |

Table 2

| | Conditioner - Dry Properties | | |
|---|---|---|---|
| | Moisturized feel | Soft Feel | Dry combing |
| Example 1 | 7.3 | 7.1 | 7.2 |
| Example 2 | 8 | 8.3 | 8.8 |
| Example 3 | 8.6 | 8.7 | 8.5 |
| Example 4 | 9.2 | 8.8 | 9.3 |

Table 3

| | Viscosity (cp) |
|---|---|
| Example 1 | 757 |
| Example 2 | 536 |
| Example 3 | 1,509 |
| Example 4 | 580 |

The dimensions and values disclosed herein are not to be understood as being strictly limited to the exact numerical values recited. Instead, unless otherwise specified, each such dimension is intended to mean both the recited value and a functionally equivalent range surrounding that value. For example, a dimension disclosed as "40 mm" is intended to mean "about 40 mm."

Every document cited herein, including any cross referenced or related patent or application and any patent application or patent to which this application claims priority or benefit thereof, is hereby incorporated herein by reference in its entirety unless expressly excluded or otherwise limited. The citation of any document is not an admission that it is prior art with respect to any invention disclosed or claimed herein or that it alone, or in any combination with any other reference or references, teaches, suggests or discloses any such invention. Further, to the extent that any meaning or

definition of a term in this document conflicts with any meaning or definition of the same term in a document incorporated by reference, the meaning or definition assigned to that term in this document shall govern.

While particular embodiments of the present invention have been illustrated and described, it would be obvious to those skilled in the art that various other changes and modifications can be made without departing from the scope of the invention. It is therefore intended to cover in the appended claims all such changes and modifications that are within the scope of this invention.

## Claims

**1.** A method of treating the hair, the method comprising:

a. providing a concentrated hair care composition in an aerosol foam dispenser, wherein the concentrated hair care composition comprises:

i. from 4% to 20%, preferably from 4.5% to 15%, preferably from 5% to 10% of one or more silicones, by weight of the concentrated hair care composition, wherein the particle size of the one or more silicones is from 1 nm to 300 nm;
ii. from 2% to 6% fatty alcohols, by weight of the concentrated hair care composition, wherein the fatty alcohols comprise stearyl alcohol and cetyl alcohol, and wherein the weight ratio of the stearyl alcohol to the cetyl alcohol is from 1.5:1 to 5:1;
iii. from 1% to 10% propellant, by weight of the concentrated hair care composition;
iv. from 0.5% to 7% perfume, by weight of the concentrated hair care composition; and
v. from 75% to 95% water, by weight of the concentrated hair care composition;

wherein the concentrated hair care composition has a liquid phase viscosity of from 200 centipoise to 15,000 centipoise;
wherein the concentrated hair care composition has a silicone to high melting point fatty compound weight ratio of from 90:10 to 40:60; and
wherein the concentrated hair care composition has a silicone to perfume weight ratio of from 98:2 to 50:50;
b. dispensing the concentrated hair care composition from the aerosol foam dispenser as a foam;
c. applying the foam to the hair; and
d. rinsing the foam from the hair;

wherein the foam has a density of from 0.025 g/$cm^3$ to 0.30 g/$cm^3$ when dispensed from the aerosol foam dispenser.

**2.** The method of Claim 1, wherein the silicone to high melting point fatty compound weight ratio is from 80:20 to 40:60, preferably from 70:30 to 50:50.

**3.** The method of Claim 1, wherein the foam comprises a silicone deposition purity of from 40% to 90%, preferably from 45% to 80% after applying the foam to the hair and rinsing the foam from the hair.

**4.** The method of Claims 1 or 2, wherein the concentrated hair care composition is a nanoemulsion.

**5.** The method of any preceding claim, wherein the concentrated hair care composition comprises from 2% to 5% high melting point fatty compounds, by weight of the concentrated hair care composition.

**6.** The method of any preceding claim, wherein the concentrated hair care composition comprises from 2% to 4% high melting point fatty compounds, by weight of the concentrated hair care composition.

**7.** The method of any preceding claim, wherein the concentrated hair care composition comprises from 2% to 3% high melting point fatty compounds, by weight of the concentrated hair care composition.

**8.** The method of any preceding claim, wherein the concentrated hair care composition comprises an alkyl quat cationic surfactant, wherein the weight ratio of the fatty alcohols to the alkyl quat cationic surfactant is of from 2.3:1 to 5:1.

**9.** The method of any preceding claim, wherein the concentrated hair care composition comprises from 1% to 6% perfume, by weight of the concentrated hair care composition.

**10.** The method of any preceding claim, wherein the foam has a dosage weight of from 1 g to 6 g after dispensing the concentrated hair care composition from the aerosol foam dispenser.

**11.** The method of any preceding claim, wherein the density of the foam is from 0.035 g/$cm^3$ to 0.20 g/$cm^3$ after dispensing the concentrated hair care composition from the aerosol foam dispenser.

**12.** The method of any preceding claim, wherein the density of the foam is from 0.05 g/$cm^3$ to 0.15 g/$cm^3$ after dispensing the concentrated hair care composition from the aerosol foam dispenser.

**13.** An aerosol foam dispenser comprising a concentrated hair care composition, the concentrated hair care composition comprising:

a. from 4% to 20% of one or more silicones, by weight of the concentrated hair care composition, wherein the particle size of the one or more silicones is from 1 nm to 300 nm;
b. from 2% to 6% fatty alcohols, by weight of the concentrated hair care composition, wherein the fatty alcohols comprise stearyl alcohol and cetyl alcohol, and wherein the weight ratio of stearyl alcohol to cetyl alcohol is from 1.5:1 to 5:1;
c. from 1% to 10% propellant, by weight of the concentrated hair care composition;
d. from 0.5% to 7% perfume, by weight of the concentrated hair care composition; and
e. from 75% to 95% water, by weight of the concentrated hair care composition;

wherein the concentrated hair care composition has a liquid phase viscosity of from 200 centipoise to 15,000 centipoise;
wherein the concentrated hair care composition has a silicone to high melting point fatty compound weight ratio of from 90:10 to 40:60; and
wherein the concentrated hair care composition has a silicone to perfume weight ratio of from 98:2 to 50:50;
wherein the foam has a density of from 0.025 g/$cm^3$ to 0.30 g/$cm^3$ when dispensed from the aerosol foam dispenser; and
wherein the concentrated hair care composition is rinse-off.

**14.** The aerosol foam dispenser of Claim 13, wherein the silicone to high melting point fatty compound weight ratio is from 80:20 to 40:60.

**15.** The aerosol foam dispenser of Claims 13 or 14, wherein the foam comprises a silicone deposition purity of from 40% to 90% after applying the foam to hair and rinsing the foam from the hair.

**Patentansprüche**

**1.** Verfahren zum Behandeln des Haars, wobei das Verfahren umfasst:

a. Bereitstellen einer konzentrierten Haarpflegezusammensetzung in einem Aerosolschaumspender, wobei die konzentrierte Haarpflegezusammensetzung umfasst:

i. zu von 4 Gew.-% bis 20 Gew.-%, vorzugsweise von 4,5 Gew.-% bis 15 Gew.-%, vorzugsweise von 5 Gew.-% bis 10 Gew.-%, ein oder mehrere Silikone bezogen auf das Gewicht der konzentrierten Haarpflegezusammensetzung, wobei die Teilchengröße des einen oder der mehreren Silikone 1 nm bis 300 nm beträgt;
ii. zu von 2 Gew.-% bis 6 Gew.-% Fettalkohole bezogen auf das Gewicht der konzentrierten Haarpflegezusammensetzung, wobei die Fettalkohole Stearylalkohol und Cetylalkohol umfassen und wobei das Gewichtsverhältnis von Stearylalkohol zu Cetylalkohol 1,5:1 bis 5:1 beträgt;
iii. zu von 1 Gew.-% bis 10 Gew.-% Treibstoff bezogen auf das Gewicht der konzentrierten Haarpflegezusammensetzung;
iv. zu von 0,5 Gew.-% bis 7 Gew.-% Duftstoff bezogen auf das Gewicht der konzentrierten Haarpflegezusammensetzung; und
v. zu von 75 Gew.-% bis 95 Gew.-% Wasser bezogen auf das Gewicht der konzentrierten Haarpflegezusammensetzung;

wobei die konzentrierte Haarpflegezusammensetzung eine Flüssigphasenviskosität von 200 Centipose bis 15.000 Centipose aufweist;
wobei die konzentrierte Haarpflegezusammensetzung ein Verhältnis von Silikon zu Fettstoff mit hohem Schmelzpunkt von 90:10 bis 40:60 aufweist; und
wobei die konzentrierte Haarpflegezusammensetzung ein Verhältnis von Silikon zu Duftstoff von 98:2 bis 50:50 aufweist;
b. Abgeben der konzentrierten Haarpflegezusammensetzung aus dem Aerosolschaumspender als ein Schaum;
c. Aufbringen des Schaums auf das Haar; und
d. Spülen des Schaums aus dem Haar;

wobei der Schaum beim Abgeben aus dem Aerosolschaumspender eine Dichte von 0,025 g/$cm^3$ bis 0,30 g/$cm^3$ aufweist.

**2.** Verfahren nach Anspruch 1, wobei das Gewichtsverhältnis von Silikon zu Fettstoff mit hohem Schmelzpunkt von 80:20 bis 40:60, vorzugsweise von 70:30 bis 50:50, beträgt.

**3.** Verfahren nach Anspruch 1, wobei der Schaum nach Aufbringen des Schaums auf das Haar und Spülen des Schaums aus dem Haar eine Silikonanlagerungsreinheit von 40 % bis 90 %, vorzugsweise von 45 % bis 80 %, umfasst.

**4.** Verfahren nach Anspruch 1 oder 2, wobei die konzentrierte Haarpflegezusammensetzung eine Nanoemulsion ist.

**5.** Verfahren nach einem der vorstehenden Ansprüche, wobei die konzentrierte Haarpflegezusammensetzung zu von 2 Gew.-% bis 5 Gew.-% Fettstoffe mit hohem Schmelzpunkt bezogen auf das Gewicht der konzentrierten Haarpflegezusammensetzung umfasst.

**6.** Verfahren nach einem der vorstehenden Ansprüche, wobei die konzentrierte Haarpflegezusammensetzung zu von 2 Gew.-% bis 4 Gew.-% Fettstoffe mit hohem Schmelzpunkt bezogen auf das Gewicht der konzentrierten Haarpflegezusammensetzung umfasst.

**7.** Verfahren nach einem der vorstehenden Ansprüche, wobei die konzentrierte Haarpflegezusammensetzung zu von 2 Gew.-% bis 3 Gew.-% Fettstoffe mit hohem Schmelzpunkt bezogen auf das Gewicht der konzentrierten Haarpflegezusammensetzung umfasst.

**8.** Verfahren nach einem der vorstehenden Ansprüche, wobei die konzentrierte Haarpflegezusammensetzung ein kationenaktives Alkylquattensid umfasst, wobei das Gewichtsverhältnis der Fettalkohole zu dem kationenaktiven Alkylquattensid 2,3:1 bis 5:1 beträgt.

**9.** Verfahren nach einem der vorstehenden Ansprüche, wobei die konzentrierte Haarpflegezusammensetzung zu von 1 Gew.-% bis 6 Gew.-% Duftstoff bezogen auf das Gewicht der konzentrierten Haarpflegezusammensetzung umfasst.

**10.** Verfahren nach einem der vorstehenden Ansprüche, wobei der Schaum nach Abgeben der konzentrierten Haarpflegezusammensetzung aus dem Aerosolschaumspender ein Dosierungsgewicht von 1 g bis 6 g aufweist.

**11.** Verfahren nach einem der vorstehenden Ansprüche, wobei die Dichte des Schaums nach Abgeben der konzentrierten Haarpflegezusammensetzung aus dem Aerosolschaumspender 0,035 g/$cm^3$ bis 0,20 g/$cm^3$ beträgt.

**12.** Verfahren nach einem der vorstehenden Ansprüche, wobei die Dichte des Schaums nach Abgeben der konzentrierten Haarpflegezusammensetzung aus dem Aerosolschaumspender 0,05 g/$cm^3$ bis 0,15 g/$cm^3$ beträgt.

**13.** Aerosolschaumspender, umfassend eine konzentrierte Haarpflegezusammensetzung, wobei die konzentrierte Haarpflegezusammensetzung umfasst:

a. zu von 4 Gew.-% bis 20 Gew.-% ein oder mehrere Silikone bezogen auf das Gewicht der konzentrierten Haarpflegezusammensetzung, wobei die Teilchengröße des einen oder der mehreren Silikone von 1 nm bis 300 nm beträgt;
b. zu von 2 Gew.-% bis 6 Gew.-% Fettalkohole bezogen auf das Gewicht der konzentrierten Haarpflegezusammensetzung, wobei die Fettalkohole Stearylalkohol und Cetylalkohol umfassen und wobei das Gewichtsverhältnis von Stearylalkohol zu Cetylalkohol von 1,5:1 bis 5:1 beträgt;
c. zu von 1 Gew.-% bis 10 Gew.-% Treibstoff bezogen auf das Gewicht der konzentrierten Haarpflegezusammensetzung;
d. zu von 0,5 Gew.-% bis 7 Gew.-% Duftstoff bezogen auf das Gewicht der konzentrierten Haarpflegezusammensetzung; und
e. zu von 75 Gew.-% bis 95 Gew.-% Wasser bezogen auf das Gewicht der konzentrierten Haarpflegezusammensetzung;

wobei die konzentrierte Haarpflegezusammensetzung eine Flüssigphasenviskosität von 200 Centipose bis 15.000 Centipose aufweist;
wobei die konzentrierte Haarpflegezusammensetzung ein Verhältnis von Silikon zu Fettstoff mit hohem Schmelzpunkt von 90:10 bis 40:60 aufweist; und
wobei die konzentrierte Haarpflegezusammensetzung ein Verhältnis von Silikon zu Duftstoff von 98:2 bis 50:50 aufweist;
wobei der Schaum beim Abgeben aus dem Aerosolschaumspender eine Dichte von 0,025 g/cm$^3$ bis 0,30 g/cm$^3$ aufweist; und
wobei die konzentrierte Haarpflegezusammensetzung zum Ausspülen ist.

**14.** Aerosolschaumspender nach Anspruch 13, wobei das Gewichtsverhältnis von Silikon zu Fettstoff mit hohem Schmelzpunkt von 80:20 bis 40:60 beträgt.

**15.** Aerosolschaumspender nach Anspruch 13 oder 14, wobei der Schaum nach Aufbringen des Schaums auf das Haar und Spülen des Schaums aus dem Haar eine Silikonanlagerungsreinheit von 40 % bis 90 % umfasst.

**Revendications**

**1.** Procédé de traitement des cheveux, le procédé comprenant :

a. la fourniture d’une composition pour soins capillaires concentrée dans un distributeur de mousse en aérosol, dans lequel la composition pour soins capillaires concentrée comprend :

i. de 4 % à 20 %, de préférence de 4,5 % à 15 %, de préférence de 5 % à 10 % d’une ou plusieurs silicones, en poids de la composition pour soins capillaires concentrée, dans lequel la taille des particules des une ou plusieurs silicones se situe dans l’intervalle allant de 1 nm à 300 nm ;
ii. de 2 % à 6 % d’alcools gras, en poids de la composition pour soins capillaires concentrée, dans lequel les alcools gras comprennent de l’alcool stéarylique et de l’alcool cétylique, et dans lequel le rapport pondéral de l’alcool stéarylique à l’alcool cétylique se situe dans l’intervalle allant de 1,5:1 à 5:1 ;
iii. de 1 % à 10 % d’agent propulseur, en poids de la composition pour soins capillaires concentrée ;
iv. de 0,5 % à 7 % de parfum, en poids de la composition pour soins capillaires concentrée ; et
v. de 75 % à 95 % d’eau, en poids de la composition pour soins capillaires concentrée ;

dans lequel la composition pour soins capillaires concentrée a une viscosité de phase liquide allant de 200 centipoise à 15 000 centipoise ;
dans lequel la composition pour soins capillaires concentrée a un rapport pondéral de la silicone au composé gras à haut point de fusion situé dans l’intervalle allant de 90:10 à 40:60 ; et
dans lequel la composition pour soins capillaires concentrée a un rapport pondéral de la silicone au parfum situé dans l’intervalle allant de 98:2 à 50:50 ;
b. la distribution de la composition pour soins capillaires concentrée à partir du distributeur de mousse en aérosol sous forme d’une mousse ;
c. l’application de la mousse sur les cheveux ; et
d. le rinçage de la mousse à partir des cheveux ;

dans lequel la mousse a une masse volumique allant de 0,025 g/cm$^3$ à 0,30 g/cm$^3$ lorsqu’elle est distribuée à partir du distributeur de mousse en aérosol.

**2.** Procédé selon la revendication 1, dans lequel le rapport pondéral de la silicone au composé gras à haut point de

fusion se situe dans l'intervalle allant de 80:20 à 40:60, de préférence de 70:30 à 50:50.

**3.** Procédé selon la revendication 1, dans lequel la mousse comprend une pureté de dépôt de silicone allant de 40 % à 90 %, de préférence de 45 % à 80 %, après l'application de la mousse sur les cheveux et le rinçage de la mousse à partir des cheveux.

**4.** Procédé selon les revendications 1 ou 2, dans lequel la composition pour soins capillaires concentrée est une nanoémulsion.

**5.** Procédé selon une quelconque revendication précédente, dans lequel la composition pour soins capillaires concentrée comprend de 2 % à 5 % de composés gras à haut point de fusion, en poids de la composition pour soins capillaires concentrée.

**6.** Procédé selon une quelconque revendication précédente, dans lequel la composition pour soins capillaires concentrée comprend de 2 % à 4 % de composés gras à haut point de fusion, en poids de la composition pour soins capillaires concentrée.

**7.** Procédé selon une quelconque revendication précédente, dans lequel la composition pour soins capillaires concentrée comprend de 2 % à 3 % de composés gras à haut point de fusion, en poids de la composition pour soins capillaires concentrée.

**8.** Procédé selon une quelconque revendication précédente, dans lequel la composition pour soins capillaires concentrée comprend un agent tensioactif cationique alkyle quaternaire, dans lequel le rapport pondéral des alcools gras à l'agent tensioactif cationique alkyle quaternaire est de 2,3:1 à 5:1.

**9.** Procédé selon une quelconque revendication précédente, dans lequel la composition pour soins capillaires concentrée comprend de 1 % à 6 % de parfum, en poids de la composition pour soins capillaires concentrée.

**10.** Procédé selon une quelconque revendication précédente, dans lequel la mousse a un poids de dosage allant de 1 g à 6 g après la distribution de la composition pour soins capillaires concentrée à partir du distributeur de mousse en aérosol.

**11.** Procédé selon une quelconque revendication précédente, dans lequel la masse volumique de la mousse se situe dans l'intervalle allant de 0,035 g/cm$^3$ à 0,20 g/cm$^3$ après la distribution de la composition pour soins capillaires concentrée du distributeur de mousse en aérosol.

**12.** Procédé selon une quelconque revendication précédente, dans lequel la masse volumique de la mousse se situe dans l'intervalle allant de 0,05 g/cm$^3$ à 0,15 g/cm$^3$ après la distribution de la composition pour soins capillaires concentrée du distributeur de mousse en aérosol.

**13.** Distributeur de mousse en aérosol comprenant une composition pour soins capillaires concentrée, la composition pour soins capillaires concentrée comprenant :

a. de 4 % à 20 % d'une ou plusieurs silicones, en poids de la composition pour soins capillaires concentrée, dans lequel la taille des particules des une ou plusieurs silicones se situe dans l'intervalle allant de 1 nm à 300 nm ;
b. de 2 % à 6 % d'alcools gras, en poids de la composition pour soins capillaires concentrée, dans lequel les alcools gras comprennent de l'alcool stéarylique et de l'alcool cétylique, et dans lequel le rapport pondéral de l'alcool stéarylique à l'alcool cétylique se situe dans l'intervalle allant de 1,5:1 à 5:1 ;
c. de 1 % à 10 % d'agent propulseur, en poids de la composition pour soins capillaires concentrée ;
d. de 0,5 % à 7 % de parfum, en poids de la composition pour soins capillaires concentrée ; et
e. de 75 % à 95 % d'eau, en poids de la composition pour soins capillaires concentrée ;

dans lequel la composition pour soins capillaires concentrée a une viscosité de phase liquide allant de 200 centipoise à 15 000 centipoise ;
dans lequel la composition pour soins capillaires concentrée a un rapport pondéral de la silicone au composé gras à haut point de fusion situé dans l'intervalle allant de 90:10 à 40:60 ; et
dans lequel la composition pour soins capillaires concentrée a un rapport pondéral de la silicone au parfum situé dans l'intervalle allant de 98:2 à 50:50 ; dans lequel la mousse a une masse volumique allant de 0,025 g/cm$^3$ à 0,30

g/$cm^3$ lorsqu'elle est distribuée à partir du distributeur de mousse en aérosol ; et
dans lequel la composition pour soins capillaires concentrée est à éliminer par rinçage.

**14.** Distributeur de mousse en aérosol selon la revendication 13, dans lequel le rapport pondéral de la silicone au composé gras à haut point de fusion se situe dans l'intervalle allant de 80:20 à 40:60.

**15.** Distributeur de mousse en aérosol selon les revendications 13 ou 14, dans lequel la mousse comprend une pureté de dépôt de silicone allant de 40 % à 90 % après l'application de la mousse sur les cheveux et le rinçage de la mousse à partir des cheveux.

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description


- US 4185087 A
- EP 0530974 A
- WO 2002010257 PCT
- US 3480616 A
- US 4565647 A, Llenado
- WO 2013148778 A
- EP 0283165 B1


### Non-patent literature cited in the description


- International Cosmetic Ingredient Dictionary. 1993
- CTFA Cosmetic Ingredient Handbook. 1992
- CTFA Cosmetic Ingredient Handbook. The Cosmetic, Toiletries, and Fragrance Association, Inc, 1988